# EUROPEAN PATENT APPLICATION

(11) **EP 4 421 173 A1**
(43) Date of publication of application: **28.08.2024**
(21) Application number: 23158617.3
(22) Date of filing: 24.02.2023
(51) Int. Cl.: C12N 15/11, A61P 11/00

(54) **USE OF MICRORNAS IN THE TREATMENT OF LUNG DISEASES**

(71) Applicant: International Centre For Genetic Engineering And Biotechnology - ICGEB, 34149 Trieste (IT)
(72) Inventor: BRAGA, Luca, 34149 Trieste (TS) (IT); VOLPE, Maria Concetta, 34149 Trieste (TS) (IT)
(74) Representative: Pace Napoleone, Maria

(57) **Abstract**

The present invention refers to an agent for use in the prevention and/or treatment of lung pathological conditions associated with loss of alveolar epithelial type II and/or type I cells and/or with loss of lung regenerative capacity and/or for use in promoting lung regeneration in a subject with a lung condition, said agent being selected from the group consisting of: hsa-miR-124-3p, hsa-miR-5008-5p, hsa-miR-4255, hsa-miR-4691-5p, hsa-miR-4781-5p,h sa-miR-4782-5p, hsa-miR-3928-3p , inhibitor of hsa-miR-183-3p, inhibitor of hsa-miR-16-2-3p, inhibitor of hsa-mir-147b-3p and inhibitor of hsa-miR-1292-3p or a combination thereof.

## Description

### FIELD OF THE INVENTION

The present invention relates to at least one agent selected from human microRNAs hsa-miR-124-3p, hsa-miR-5008-5p, hsa-miR-4781-5p, hsa-miR-4782-5p , hsa-miR-4691-5p, hsa-miR-4255, hsa-miR-3928-3p or a precursor or a synthetic mimic or a functional derivative thereof and/or from inhibitors of hsa-miR-183-3p, hsa-miR-16-2-3p, hsa-miR-147b-3p, hsa-miR-1292-3p or a combination thereof for use in the treatment and/or prevention of lung pathological conditions associated with loss of alveolar epithelial type II and/or type I cells and/or with loss of lung regenerative capacity and/or for use in promoting lung regeneration in a subject with a lung condition, wherein the pathological condition is preferably selected from the group of interstitial lung diseases, preferably idiopathic pulmonary fibrosis, non-specific interstitial pneumonia, desquamative interstitial pneumonia, respiratory bronchiolitis-ILD, cryptogenic organizing pneumonia, acute interstitial pneumonia, chronic obstructive pulmonary diseases, acute and post-acute respiratory distress syndrome, post-acute COVID-19 syndrome.

### BACKGROUND OF THE INVENTION

The lung is an organ that, due to its direct connection to the external environment, maintained an intrinsic regenerative capacity. The core functional structures in the lung are alveoli, the distal region of the organ where gas-exchange and blood oxygenation occur. Independently from the type of insult, either unknown (idiopathic) or known (environmental stressors, genetic disorders, infective agents (viruses, bacteria and fungi), mechanical stresses, toxic and/or irritant agents, and traumatic injuries), alveolar damage usually evolves into two directions:
1) proper alveolar repair leading to preserved organ function;
2) improper alveolar repair leading to progressive fibrosing interstitial lung disease and impaired lung function.

Chronic progressive Interstitial Lung Diseases (ILDs) with either known or unknown (Idiopathic) cause are characterized by gradual fibrotic scarring of the lungs that leads to the anatomopathological and radiological pattern of usual interstitial pneumonia (UIP) and, eventually, to death. Idiopathic Pulmonary Fibrosis (IPF) represents the most common encountered ILD in clinical practice, ranging from 0.9 to 14 cases/100.000/y in incidence worldwide [1]. At the cellular level, pulmonary fibrosis can be considered as a compensatory response to impaired lung regeneration following alveolar injury and leading to fibrotic scarring. Several studies suggest a central role of alveolar type II epithelial cells (ATII) cells in lung regeneration [2-4]. Physiologically, the turnover of alveolar type I epithelial cells (ATI) cells is supported by the trans-differentiation of the type II alveolar epithelial ATII) cells into ATI cells; ATII cells are also responsible for surfactant production [3]. In particular, it has been shown that both extensive ATII loss or pathological "activation" of ATII cells, after lung injury, may trigger inadequate repair and onset of IPF [3][5-10].

MicroRNAs (miRNAs or miRs) are evolutionarily conserved small noncoding RNAs that regulate gene expression at the post-transcriptional level. In animal cells, repression of gene expression by microRNAs is achieved by base-pairing to partially complementary sequences present mostly in 3' UTRs of target messenger RNAs (mRNAs), resulting in translation repression, mRNA degradation, or both [11]. The microRNA seed sequence, essential for the binding of the microRNA to the mRNA, is a conserved sequence, which is generally situated at positions 2-8 from the microRNA 5'-end [12]. Even though base pairing of microRNA and its target mRNA is not perfect, the region corresponding to the seed sequence usually has to be perfectly complementary. Thus, the seed sequence is the primary specificity determinant for target selection. The small size of the seed sequence means that a single microRNA can regulate many, even hundreds, different genes. MicroRNAs are genome-encoded sequences generally transcribed by RNA polymerase II into primary microRNAs (pri-microRNAs). The pri-microRNAs are then sequentially processed by two endonucleases of the RNAse III family: in the nucleus, Drosha processes the pri-microRNA into a precursor microRNA (pre-microRNA) of approximately 60-80 nucleotides, after which the pre-microRNA is further processed, in the cytoplasm, by Dicer to form a duplex containing two strands, of about 19-23 nucleotides. The microRNA duplex is then unwound, and the mature microRNA is incorporated into the RNA-induced silencing complex (RISC), containing, among others, Argonaute and GW182 proteins essential for the silencing by microRNAs [13].

MicroRNAs perform relevant roles in maintaining lung functions and homeostasis [14]. They are involved in several cellular processes which occur in lung, such as cell proliferation, cell differentiation, apoptosis, and inflammatory responses. The importance of miRNAs in lung development has been demonstrated by Harris et al., using hybridization experiments *in situ* [15]. They showed that conditional knock out of Dicer1 in the murine lung epithelium, in both neonatal and adult lung, leads to an improper branching morphogenesis and a severe ECM production with fibrosis development [15]. Several miRNAs have been discovered to regulate specific processes in lung repair, such as miR-26, miR-142, miR-17, miR-92 that play crucial role in ATI and ATII cell homeostasis [16]. On the contrary, there are several miRNAs that have been involved in the pathogenesis of lung diseases, such as inflammation and viral infections, lung carcinogenesis, pulmonary allergy, asthma, chronic obstructive pulmonary disease and IPF [17].

It has been estimated that about 10% of microRNAs are dysregulated in IPF, suggesting their importance in the pathogenesis of IPF [18]. To date, it has been showed that the deregulation of miRNAs could induce epithelial-mesenchymal transition (EMT) in alveolar epithelial cells, cell senescence, abnormal transition of fibroblast in myofibroblast with deposition of ECM in the lungs [19]. Specifically, Let-7, miR-26a, miR-200, miR-326 and miR-1343, essential for the regulation of EMT in the alveolar epithelial cells, are downregulated in IPF lungs [20].

Several studies showed that miR-200a, miR-200b and miR-200c are both downregulated in mice and in IPF patients ([21], [22], [23]). MiR-200 family and its targets are essential during lung development, as they suppress several pathways including TGF-beta, Wnt and Notch [24]. It has been demonstrated that these pathways have pivotal role in human repair and differentiation. However, they are completely altered in both murine and human fibrotic lungs [21].

Based on this evidence, inventors tested the effect of various members of the microRNA-200 family (hsa-miR-200a-3p, hsa-miR-141-3p, hsa-miR-200b-3p, hsa-miR-200c-3p), a miRNA family known to be down-regulated in IPF patients, on primary human ATII purified from IPF patients. Inventors' results showed that hsa-miR-200b-3p and miR-200c-3p were effective in promoting trans-differentiation of ATII into ATI cells [25], thus suggesting that microRNAs could be exploited as a therapeutical agent.

Other microRNAs such as miR-21, miR-29, miR-153 are abnormally regulated in IPF lungs [26]. In normal lung, miR-21 is downregulated, leading to upregulation of Smad-7. Smad-7 inhibits the proliferation of interstitial fibroblasts. But in IPF lung, there is an increase in miR-21 expression, thus an altered control of smad-7 [27]. MicroRNA-29 is decreased in the IPF lung and the restoration of miR-29 physiological levels showed protective effect in mouse model of pulmonary fibrosis [17]. MicroRNA-29 has been shown to negatively regulate the production of collagen, thus its downregulation causes excessive ECM deposition, contributing to the development of fibrosis [28]. Another miRNA involved the development of IPF is miR-153. Normally, this miRNA targets TGF-β1, regulating the proliferation and activation of fibroblasts and the ECM deposition. However it is down expressed in fibrotic lung [29].

MicroRNA-based therapeutic approaches can be subdivided into two fundamental groups: microRNA replacement and miRNA inhibition [30]. The first system is used to rescue the microRNA effect, decreasing the expression of specific microRNA target. The second approach takes advantage of modified DNA molecules called Locked Nucleic Acids (LNA), used to decrease the microRNA expression based on sequence complementarity, thus restoring the correct level of the dysregulated miRNA [31].

In the last years, different carriers for miRNAs are developed, such as liposomes, lipid nanoparticles (LNPs), extracellular vehicles (EVs), macrovesicles and viral vectors [32]. Liposomes mimic the composition of cell membrane. Liposomes are subdivided into neutral, anionic and cationic types, for example Polyethyleneglycol (PEG) lipids are widely used [33]. However, they can cause undesired immune responses and toxicity. LNPs are mainly used for *in vivo* delivery of miRNAs due to their numerous advantages, such as payload preservation of miRNA agents, improvement of target specificity and tissue distribution [34]. Finally, viral vectors are used to deliver genetic material. Among viral vectors developed for gene delivery in humans there are adeno-associated viruses (AAVs) [35]. Currently, Adeno-associated virus (AAV) vectors are the leading platform for the delivery of genetic material for the treatment of a variety of human diseases and represent valuable alternative for the delivery of miRNAs to the lung.

### SUMMARY OF THE INVENTION

Progressive fibrosing interstitial lung disease occurs when alveolar regeneration is impaired due to the inability of alveolar type II cells (ATIIs) to trans-differentiate into alveolar type I cells (ATIs). Therefore, ATII to ATI trans-differentiation process represents a relevant target for the development of innovative regenerative therapy for the human lung. In this perspective, inventor phenotypically screened 2042 human microRNAs on primary murine ATIIs looking for microRNAs regulating ATIIs to ATIs trans-differentiation. Following this approach, they selected 7 miRNAs promoting and 4 miRNAs blocking ATIIs to ATIs trans-differentiation. All 11 selected microRNAs were further characterized and resulted safe by showing no pro-tumorigenic effect *in vitro* on A549 cells. In addition, three of the selected microRNAs (hsa-miR-124-3p, hsa-miR-4781-5p, hsa-miR-147b-3p) showed an antifibrotic effect *in vitro* on primary alveolar lung fibroblast by both reducing fibroblast proliferation and fibroblast to myofibroblast activation.

These data suggests that the selected microRNAs could represent viable candidates for the development of novel therapies aimed at both restoring the physiological regenerative capacity of the human lungs and therefore blunting the aberrant fibrotic response usually occurring after chronic and acute alveolar damage.

The present inventors therefore found that the human microRNAs: hsa-miR-124-3p, hsa-miR-5008-5p, hsa-miR-4781-5p, hsa-miR-4782-5p , hsa-miR-4691-5p, hsa-miR-4255, hsa-miR-3928-3p, and inhibitors of hsa-miR-183-3p, hsa-miR-16-2-3p, hsa-miR-147b-3p, hsa-miR-1292-3p are effective for the treatment of lung diseases associated with loss of alveolar epithelial type II and type I cells and consequent pathological remodelling of the lung, in particular for preventing and/or treating IPF. Inventors here propose, among others, the use of selected human microRNAs formulated either as synthetic miRNA mimics or as recombinant viral vectors, as e.g. AAV6.2FF vectors, expressing the genetic information encoding for the selected miRNA sequences or the use of inhibitors of selected microRNAs to push the reprogramming of ATII cells into ATI cells as a novel therapeutical strategy to promote lung regeneration after alveolar injury.

It is therefore an object of the invention an agent for use in the prevention and/or treatment of lung pathological conditions associated with loss of alveolar epithelial type II and/or type I cells and/or with loss of lung regenerative capacity and/or for use in promoting lung regeneration in a subject with a lung condition, said agent being selected from the group consisting of:
- hsa-miR-124-3p or a primary transcript or a precursor or a synthetic mimic or a functional derivative thereof;
- hsa-miR-5008-5p or a primary transcript or a precursor or a synthetic mimic or a functional derivative thereof;
- hsa-miR-4255 or a primary transcript or precursor or a synthetic mimic or a functional derivative thereof;
- hsa-miR-4691-5p or a primary transcript or a precursor or a synthetic mimic or a functional derivative thereof;
- hsa-miR-4781-5p or a primary transcript or a precursor or a synthetic mimic or a functional derivative thereof;
- hsa-miR-4782-5p or a primary transcript or a precursor or a synthetic mimic or a functional derivative thereof;
- hsa-miR-3928-3p or a primary transcript or a precursor or a synthetic mimic or a functional derivative thereof;
- inhibitor of hsa-miR-183-3p;
- inhibitor of hsa-miR-16-2-3p;
- inhibitor of hsa-mir-147b-3p;
- inhibitor of hsa-miR-1292-3p;
or a combination thereof.

Preferably the pathological condition is selected from the group consisting of: interstitial lung diseases, preferably idiopathic pulmonary fibrosis, non-specific interstitial pneumonia, desquamative interstitial pneumonia, respiratory bronchiolitis-ILD, cryptogenic organizing pneumonia, acute interstitial pneumonia, chronic obstructive pulmonary diseases, acute and post-acute respiratory distress syndrome, post-acute COVID-19 syndrome.

Preferably the lung condition is selected from the group consisting of: interstitial lung diseases, preferably idiopathic pulmonary fibrosis, non-specific interstitial pneumonia, desquamative interstitial pneumonia, respiratory bronchiolitis-ILD, cryptogenic organizing pneumonia, acute interstitial pneumonia, chronic obstructive pulmonary diseases, acute and post-acute respiratory distress syndrome, post-acute COVID-19 syndrome.

Preferably the pathological condition or the lung condition is idiopathic pulmonary fibrosis, chronic obstructive pulmonary diseases, acute and post-acute respiratory distress syndrome.

Preferably, said inhibitor is a nucleic acid molecule preferably selected from: a single-stranded or double-stranded nucleic acid molecule, an siRNA molecule, an antisense oligonucleotide, derivatives and mixtures thereof.

Preferably, said nucleic acid molecule or inhibitor has sufficient complementarity to hsa-miR-183-3p, hsa-miR-16-2-3p, hsa-mir-147b-3p or hsa-miR-1292-3p to form a hybrid under physiological conditions and/or comprises at least 8 nucleotides complementary to at least one sequence selected from SEQ ID NO: 17-24 or a variant thereof.

Preferably the inhibitor of hsa-miR-183-3p comprises a nucleotide sequence comprising 5'-GTAATTCA-3' and/or the inhibitor comprises about 8 to about 30 nucleotides in length.

Preferably, the inhibitor of hsa-miR-16-2-3p comprises a nucleotide sequence comprising 5'-TAATATTG'-3' and/or the inhibitor comprises about 8 to about 30 nucleotides in length. Preferably, the inhibitor of hsa-mir-147b-3p comprises a nucleotide sequence comprising 5'-TCCGCACA-3' and/or the inhibitor comprises about 8 to about 30 nucleotides in length. Preferably, the inhibitor of hsa-miR-1292-3p comprises a nucleotide sequence comprising 5'-GGGGCGCG-3' and/or the inhibitor comprises about 8 to about 30 nucleotides in length. Preferably, the inhibitor comprises at least 1 nucleotide, at least 2 nucleotides, at least 3 nucleotides, at least 4 nucleotides, at least 5 nucleotides, at least 6 nucleotides, at least 7 nucleotides, at least 8 nucleotides, at least 9 nucleotides, at least 10 nucleotides, at least 11 nucleotides, at least 12 nucleotides, at least 13 nucleotides, at least 14 nucleotides, at least 15 nucleotides, at least 16 nucleotides, at least 17 nucleotides, at least 18 nucleotides, at least 19 nucleotides, or at least 20 nucleotides at the 5' and/or at the 3' of said nucleotide sequence.

Preferably, the inhibitor of hsa-miR-183-3p has at least 50%, 60%, 70%, 80%, 90%, 95%, 99% or 100% complementarity to a portion of a hsa-miR-183-3p sequence.

Preferably, the inhibitor of hsa-miR-16-2-3p has at least 50%, 60%, 70%, 80%, 90%, 95%, 99% or 100% complementarity to a portion of a hsa-miR-16-2-3p sequence.

Preferably, the inhibitor of hsa-mir-147b-3p and/or has at least 50%, 60%, 70%, 80%, 90%, 95%, 99% or 100% complementarity to a portion of a hsa-miR-147b-3p sequence.

Preferably, the inhibitor of hsa-miR-1292-3p has at least 50%, 60%, 70%, 80%, 90%, 95%, 99% or 100% complementarity to a portion of a hsa-miR-1292-3p sequence.

Preferably, the inhibitor binds to a miRNA comprising a nucleic acid that is at least 50%, 60%, 70%, 80%, 90%, 95%, 99% or 100% identical to any one SEQ ID NOs: 17-24.

Preferably, the inhibitor comprises at least one modified nucleotide, more preferably the at least one modified nucleotide is a locked nucleic acid (LNA), an unlocked nucleic acid (UNA), an arabino nucleic acid (ABA), a bridged nucleic acid (BNA), and/or a peptide nucleic acid (PNA).

Preferably, the inhibitor comprises a backbone modification, more preferably a phosphorodiamidate morpholino oligomer (PMO) and/or phosphorothioate (PS) modification.

Another object of the invention is a nucleic acid coding for the at least one agent as defined herein for use in the treatment and/or prevention of lung pathological conditions associated with loss of alveolar epithelial type II and/or type I cells and/or with loss of lung regenerative capacity and/or for use in promoting lung regeneration in a subject with a lung condition.

Preferably, the pathological condition or lung condition is selected from the group of interstitial lung diseases, preferably idiopathic pulmonary fibrosis, non-specific interstitial pneumonia, desquamative interstitial pneumonia, respiratory bronchiolitis-ILD, cryptogenic organizing pneumonia, acute interstitial pneumonia, chronic obstructive pulmonary diseases, acute and post-acute respiratory distress syndrome, post-acute COVID-19 syndrome.

Preferably, the agent or nucleic acid is provided naked or within a delivery agent.

Preferably, the delivery agent comprises a vector, preferably a recombinant expression vector or a viral vector, a micelle, an exosome, a lipidoid, a lipoplex, an extracellular vesicle, a synthetic vesicle, a polymeric compound, a peptide, a protein, a cell, a nanoparticle mimic, a nanotube, a conjugate, nanoparticles, microparticles, a liposome or other biological or synthetic vesicle or material including lipid nanoparticles, polymer-based nanoparticles, polymer-lipid hybrid a nanoparticle, a microparticle, a microsphere, a liposome, a colloidal gold particle, a graphene composite, a cholesterol conjugate, a cyclodextran complexe, a polyethylenimine polymer, a lipopolysaccharide, a polypeptide, a polysaccharide, a lipopolysaccharide, a collagen or a pegylation of viral vehicle, or combinations thereof.

It is another object of the invention a vector, preferably a recombinant expression vector, comprising a coding sequence for the agent as defined herein or the nucleic acid as defined herein, and/or expressing the agent as defined herein or the nucleic acid as defined herein, preferably under the control of a suitable promoter, for use in the treatment and/or prevention of lung pathological conditions associated with loss of alveolar epithelial type II and/or type I cells and/or with loss of lung regenerative capacity and/or for use in promoting lung regeneration in a subject with a lung condition, wherein the pathological condition is preferably selected from the group of interstitial lung diseases, preferably idiopathic pulmonary fibrosis, non-specific interstitial pneumonia, desquamative interstitial pneumonia, respiratory bronchiolitis-ILD, cryptogenic organizing pneumonia, acute interstitial pneumonia, chronic obstructive pulmonary diseases, acute and post-acute respiratory distress syndrome, post-acute COVID-19 syndrome.

Preferably, the vector is a viral or non-viral vector, more preferably the viral vector is selected from adeno-associated virus (AAV) vectors of any capsid serotype, either natural or artificial, lentivirus vectors, adenoviral vector, retroviral vectors, alphaviral vectors, vaccinia virus vectors, herpes simplex virus (HSV) vectors, rabies virus vectors, and Sindbis virus vectors. Preferably the adeno-associated virus (AAV) vector is AAV6, AAV6.2 or AAV6.2FF.

A further object of the invention is a host cell transformed with the vector as defined herein for use in the treatment and/or prevention of lung pathological conditions associated with loss of alveolar epithelial type II and/or type I cells and/or with loss of lung regenerative capacity and/or for use in promoting lung regeneration in a subject with a lung condition, wherein the pathological condition is preferably selected from the group of interstitial lung diseases, preferably idiopathic pulmonary fibrosis, non-specific interstitial pneumonia, desquamative interstitial pneumonia, respiratory bronchiolitis-ILD, cryptogenic organizing pneumonia, acute interstitial pneumonia, chronic obstructive pulmonary diseases, acute and post-acute respiratory distress syndrome, post-acute COVID-19 syndrome.

Another object of the invention is a recombinant adeno-associated virus (rAAV) particle comprising a nucleic acid encoding the at least one agent as defined herein, preferably the particle comprises a capsid derived from adeno-associated vectors AAV6, AAV6.2, AAV6.2FF, AAV8, AAV1, AAV2, AAV5, or AAV9, preferably wherein the nucleic acid is operably linked to a viral promoter or a tissue specific promoter for use in the treatment and/or prevention of lung pathological conditions associated with loss of alveolar epithelial type II and/or type I cells and/or with loss of lung regenerative capacity and/or for use in promoting lung regeneration in a subject with a lung condition, wherein the pathological condition is preferably selected from the group of interstitial lung diseases, preferably idiopathic pulmonary fibrosis, non-specific interstitial pneumonia, desquamative interstitial pneumonia, respiratory bronchiolitis-ILD, cryptogenic organizing pneumonia, acute interstitial pneumonia, chronic obstructive pulmonary diseases, acute and post-acute respiratory distress syndrome, post-acute COVID-19 syndrome.

It is an object of the present invention a pharmaceutical composition for use in the treatment and/or prevention of lung pathological conditions associated with loss of alveolar epithelial type II and/or type I cells and/or with loss of lung regenerative capacity and/or for use in promoting lung regeneration in a subject with a lung condition, wherein the pathological condition is preferably selected from the group of interstitial lung diseases, preferably idiopathic pulmonary fibrosis, non-specific interstitial pneumonia, desquamative interstitial pneumonia, respiratory bronchiolitis-ILD, cryptogenic organizing pneumonia, acute interstitial pneumonia, chronic obstructive pulmonary diseases, acute and post-acute respiratory distress syndrome, post-acute COVID-19 syndrome, said pharmaceutical composition comprising an agent as defined herein, or the nucleic acid as defined herein, or the vector as defined herein, or the host cell as defined herein, or a recombinant adeno-associated virus (rAAV) particle as defined herein and at least one pharmaceutically acceptable vehicle and/or excipient.

### DETAILED DESCRIPTION OF THE INVENTION

Inventors performed an unbiassed phenotypic screening of all human miRNAs in primary mouse ATII cells with the aim of identifying human miRNAs able to promote or inhibit ATII to ATI trans-differentiation. This is the first unbiased phenotypic screening of human miRNA mimics in primary ATII cells ever performed and will generated a unique dataset for the identification of novel druggable targets for the development of innovative therapies aimed at restoring physiological lung function after lung injury.

Currently there are no available treatments that specifically promote lung regeneration by boosting ATII to ATI trans-differentiation and the present therapeutical approach is based on the idea of reactivating the endogenous alveolar regenerative capacity rather than targeting fibrosis, which is indeed the clinical manifestation of the impossibility of the organ to regenerate.

The agents of the invention are able to reprogram ATII into ATI and/or to promote and/or increase ATII to ATI trans-differentiation.

The sequence of hsa-miR-124-3p is available in the state of the art with the ID hsa-miR-124-3p (www.mirbase.org; Database Release 21; stem-loop sequence accession number MI0000443, MI0000444 and, MI0000445, mature sequence accession number MIMAT0000422).

The sequence of hsa-miR-5008-5p is available in the state of the art with the ID hsa-miR-5008-5p (www.mirbase.org; Database Release 21; stem-loop sequence accession number MI0017876, mature sequence accession number MIMAT0021039).

The sequence of hsa-miR-4255 is available in the state of the art with the ID hsa-miR-4255 (www.mirbase.org; Database Release 21; stem-loop sequence accession number MI0015863, mature sequence accession number MIMAT0016885).

The sequence of hsa-miR-4691-5p is available in the state of the art with the ID hsa-miR-4691-5p (www.mirbase.org; Database Release 21; stem-loop sequence accession number MI0017324, mature sequence accession number MIMAT0019781).

The sequence of hsa-miR-4781-5p is available in the state of the art with the ID hsa-miR-4781-5p (www.mirbase.org; Database Release 21; stem-loop sequence accession number MI0017426, mature sequence accession number MIMAT0019942).

The sequence of hsa-miR-4782-5p is available in the state of the art with the ID hsa-miR-4782-5p (www.mirbase.org; Database Release 21; stem-loop sequence accession number MI0017427, mature sequence accession number MIMAT0019944).

The sequence of hsa-miR-3928-3p is available in the state of the art with the ID hsa-miR-3928-3p (www.mirbase.org; Database Release 21; stem-loop sequence accession number MI0016438, mature sequence accession number MIMAT0018205).

The sequence of hsa-miR-183-3p is available in the state of the art with the ID hsa-miR-183-3p (www.mirbase.org; Database Release 21; stem-loop sequence accession number MI0000273, mature sequence accession number MIMAT0004560).

The sequence of hsa-miR-16-2-3p is available in the state of the art with the ID hsa-miR-16-2-3p (www.mirbase.org; Database Release 21; stem-loop sequence accession number MI0000115, mature sequence accession number MIMAT0004518).

The sequence of hsa-mir-147b-3p is available in the state of the art with the ID hsa-mir-147b-3p (www.mirbase.org; Database Release 21; stem-loop sequence accession number MI0005544, mature sequence accession number MIMAT0004928).

The sequence of hsa-miR-1292-3p is available in the state of the art with the ID hsa-miR-1292-3p (www.mirbase.org; Database Release 21; stem-loop sequence accession number MI0006433, mature sequence accession number MIMAT0022948).

In particular, hsa-miR-124-3p can have anyone of the following sequences: 
5'AGGCCUCUCUCUCCGUGUUCACAGCGGACCUUGAUUUAAAUGUCCAUACAAUUAA
GGCACGCGGUGAAUGCCAAGAAUGGGGCUG3' (SEQ ID NO: 1)
 corresponding to the accession number MI0000443; 
5’AUCAAGAUUAGAGGCUCUGCUCUCCGUGUUCACAGCGGACCUUGAUUUAAUGUCA
UACAAUUAAGGCACGCGGUGAAUGCCAAGAGCGGAGCCUACGGCUGCACUUGAA3’
 (SEQ ID NO: 2) corresponding to the accession number MI0000444; 
5’UGAGGGCCCCUCUGCGUGUUCACAGCGGACCUUGAUUUAAUGUCUAUACAAUUAA
GGCACGCGGUGAAUGCCAAGAGAGGCGCCUCC3’ (SEQ ID NO: 3)
 corresponding to the accession number MI0000445;
5'UAAGGCACGCGGUGAAUGCCAA 3' (SEQ ID NO: 4)
corresponding to the mature sequence accession number MIMAT0000422.

In particular, hsa-miR-5008-5p can have anyone of the following sequences: 
5'GGGCUGACCCCUAGGGUCAGGUGAGGCCCUUGGGGCACAGUGGUGCCAUCUCCCC
UGUGCUCCCAGGGCCUCGCCUGUCCCUUGAGGUCGGCCC3' (SEQ ID NO: 5)
 corresponding to the accession number MI0017876;
5'UGAGGCCCUUGGGGCACAGUGG3' (SEQ ID NO: 6)
corresponding to the mature sequence accession number MIMAT0021039.

In particular, hsa-miR-4255 can have anyone of the following sequences: 
5’GAGCAUCCUUCAGUGUUCAGAGAUGGAGUCAGUAUUGGUCUGGCCAUUUUUAGGG
CAAAGAGGCAGCAUCAU3’ (SEQ ID NO: 7)
 corresponding to the accession number MI0015863;
5'CAGUGUUCAGAGAUGGA3' (SEQ ID NO: 8)
corresponding to the mature sequence accession number MIMAT0016885.

In particular, hsa-miR-4691-5p can have anyone of the following sequences: 
5'GGAGCACUCCCAGGUCCUCCAGGCCAUGAGCUGCGGCCCUGAUGUCUCUACUCCAG
CCACGGACUGAGAGUGCAUAGGAGUGUCC3' (SEQ ID NO: 9)
 corresponding to the accession number MI0017324;
5'GUCCUCCAGGCCAUGAGCUGCGG3' (SEQ ID NO: 10)
corresponding to the mature sequence accession number MIMAT0019781.

In particular, hsa-miR-4781-5p can have anyone of the following sequences: 
5'AGGUGCACGCUCUAGCGGGGAUUCCAAUAUUGGGCCAAUUCCCCCAAUGUUGGAA
UCCUCGCUAGAGCGUGCACUU3' (SEQ ID NO: 11)
 corresponding to the accession number MI0017426;
5'UAGCGGGGAUUCCAAUAUUGG3' (SEQ ID NO: 12)
corresponding to the mature sequence accession number MIMAT0019942.

In particular, hsa-miR-4782-5p can have anyone of the following sequences: 
5'AUUGCCCAGUUCUGGAUAUGAAGACAAUCAAGAAAAGAUUUGGUGUUCUUGAUU
GUCUUCAUAUCUAGAACUGGGCAGU3' (SEQ ID NO: 13)
 corresponding to the accession number MI0017427;
5'UUCUGGAUAUGAAGACAAUCAA3' (SEQ ID NO: 14)
corresponding to the mature sequence accession number MIMAT0019944.

In particular, hsa-miR-3928-3p can have anyone of the following sequences: 
5’GCUGAAGCUCUAAGGUUCCGCCUGCGGGCAGGAAGCGGAGGAACCUUGGAGCUUC
GGC -3’ (SEQ ID NO: 15)
 corresponding to the accession number MI0016438;
5'- GGAGGAACCUUGGAGCUUCGGC -3' (SEQ ID NO: 16)
corresponding to the mature sequence accession number MIMAT0018205.

In particular, hsa-miR-183-3p can have anyone of the following sequences: 
5'CCGCAGAGUGUGACUCCUGUUCUGUGUAUGGCACUGGUAGAAUUCACUGUGAACA
GUCUCAGUCAGUGAAUUACCGAAGGGCCAUAAACAGAGCAGAGACAGAUCCACGA-3'
(SEQ ID NO: 17)
 corresponding to the accession number MI0000273;
5'- GUGAAUUACCGAAGGGCCAUAA -3' (SEQ ID NO: 18)
corresponding to the mature sequence accession number MIMAT0004560.

In particular, hsa-miR-16-2-3p can have anyone of the following sequences: 
5'GUUCCACUCUAGCAGCACGUAAAUAUUGGCGUAGUGAAAUAUAUAUUAAACACCA
AUAUUACUGUGCUGCUUUAGUGUGAC -3' (SEQ ID NO: 19)
 corresponding to the accession number MI0000115;
5'- CCAAUAUUACUGUGCUGCUUUA -3' (SEQ ID NO: 20)
corresponding to the mature sequence accession number MIMAT0004518.

In particular, hsa-mir-147b-3p can have anyone of the following sequences: 
5’UAUAAAUCUAGUGGAAACAUUUCUGCACAAACUAGAUUCUGGACACCAGUGUGCG
GAAAUGCUUCUGCUACAUUUUUAGG -3’ (SEQ ID NO: 21)
 corresponding to the accession number MI0005544;
5'- GUGUGCGGAAAUGCUUCUGCU -3' (SEQ ID NO: 22)
corresponding to the mature sequence accession number MIMAT0004928.

In particular, hsa-miR-1292-3p can have anyone of the following sequences: 
5'CCUGGGAACGGGUUCCGGCAGACGCUGAGGUUGCGUUGACGCUCGCGCCCCGGCU
CCCGUUCCAGG-3' (SEQ ID NO: 23)
 corresponding to the accession number MI0006433;
5'- UCGCGCCCCGGCUCCCGUUC -3' (SEQ ID NO: 24)
corresponding to the mature sequence accession number MIMAT0022948.

Anyone of the above sequences can be used according to the present invention.

Preferably, in the present invention:
- hsa-miR-124-3p comprises or consists of the SEQ ID NO: 4 and/or
- hsa-miR-5008-5p comprises or consists of the SEQ ID NO: 6 and/or
- hsa-miR-4255 comprises or consists of the SEQ ID NO: 8 and/or
- hsa-miR-4691-5p comprises or consists of the SEQ ID NO: 10 and/or
- hsa-miR-4781-5p comprises or consists of the SEQ ID NO: 12 and/or
- hsa-miR-4782-5p comprises or consists of the SEQ ID NO: 14 and/or
- hsa-miR-3928-3p comprises or consists of the SEQ ID NO: 16 and/or
- hsa-miR-183-3p comprises or consists of the SEQ ID NO: 18 and/or
- hsa-miR-16-2-3p comprises or consists of the SEQ ID NO: 20 and/or
- hsa-mir-147b-3p comprises or consists of the SEQ ID NO: 22 and/or
- hsa-miR-1292-3p comprises or consists of the SEQ ID NO: 24.

The hsa-mirs or miRs or miRNAs mentioned in the present invention preferably comprise or consist of the herein indicated sequences. They also may comprise homologs, analogs and orthologues thereof, primary miRNA molecules, precursor miRNA molecules, mature miRNA molecules, miRNA mimetics and DNA molecules encoding said molecules. DNA molecules encoding the miRNA, pre-miRNA, pri-miRNA molecules, homologs, analogs or orthologues thereof, mature miRNA molecules, miRNA mimetics are also encompassed by the invention. The nucleic acids may be selected from RNA, DNA or nucleic acid analog molecules, e.g. as sugar- or backbone-modified ribonucleotides or deoxyribonucleotides, peptide nucleic acids (PNA) or locked nucleic acids (LNA). In the present invention any combination of the above agents may be used. E.g. hsa-miR-124-3p and hsa-miR-5008-5p or hsa-miR-124-3p, hsa-miR-5008-5p and hsa-miR-4255 can be used. More than one agent may be used.

A "miRNA inhibitor" or "inhibitor of" a miR as used herein, refers to a compound that can decrease, alter, and/or modulate miRNA expression, function, and/or activity. The miRNA inhibitor can be a polynucleotide sequence that is at least partially complementary to the target miRNA nucleic acid sequence, such that the miRNA inhibitor hybridizes to the target miRNA sequence. For instance, in some aspects, a miRNA inhibitor of the present disclosure comprises a nucleotide sequence encoding a nucleotide molecule that is at least partially complementary to the target miR nucleic acid sequence, such that the miRNA inhibitor hybridizes to the miR sequence. In further aspects, the hybridization of the miRNA inhibitor to the miR sequence decreases, alters, and/or modulates the expression, function, and/or activity of the miR. "miRNA inhibitor" may refer to a nucleotide sequence that is a reverse complement of a mature miRNA (the target site). In some embodiments, the miRNA inhibitor is chemically synthesized and/or is chemically modified to prevent nucleic acid-protein complex-induced cleavage (e.g., RISC-induced cleavage) of miRNA, enhance binding affinity to the target site, and/or provide resistance to nucleolytic degradation of the miRNA inhibitor. As used herein, an miRNA inhibitor includes any natural or artificial RNA transcripts that sequestrate miRNAs and decrease or eliminate their effects. Included herein are miRNA inhibitors that are identical to competing endogenous RNAs (ceRNAs). ceRNAs are natural and intracellular miRNA inhibitors that compete to bind to shared miRNA recognition elements (MREs) to decrease microRNA availability and relieve the repression of target RNAs.

In some embodiments, the inhibitor comprises or consist of a nucleic acid complementary to any one of SEQ ID NOs: 17-24.

The terms "miRNA", "miR" and "microRNA" are used interchangeably and refer to a microRNA molecule found in eukaryotes that is involved in RNA-based gene regulation. Such terms refer to the single-stranded RNA molecule processed from a precursor. In some aspects, the term "antisense oligomers" can also be used to describe the microRNA molecules of the present disclosure.

MicroRNAs recognize and bind to target mRNAs through imperfect base pairing leading to destabilization or translational inhibition of the target mRNA and thereby downregulate target gene expression. Conversely, targeting miRNAs via molecules comprising a miRNA binding site (generally a molecule comprising a sequence complementary to the seed region of the miRNA) can reduce or inhibit the miRNA-induced translational inhibition leading to an upregulation of the target gene.

In some aspects, a miR inhibitor of the present invention comprises a nucleotide sequence encoding a nucleotide molecule that comprises at least one miR binding site, wherein the nucleotide molecule does not encode a protein. In some aspects, the miR binding site is at least partially complementary to the target miRNA nucleic acid sequence, such that the miR inhibitor hybridizes to the miR nucleic acid sequence. In some aspects, the miR binding site of a miR inhibitor disclosed herein has at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, at least about 99%, or about 100% sequence complementarity to the nucleic acid sequence of the miR. In certain aspects, the miR binding site is fully complementary to the nucleic acid sequence of the miR.

In certain aspects, the miR binding site is complementary to the miR except for 1, 2, 3, 4, 5, 6, 7, 8, 9, or 10 mismatches.

The seed region of a miRNA forms a tight duplex with the target mRNA. Most miRNAs imperfectly base-pair with the 3' untranslated region (UTR) of target mRNAs, and the 5' proximal "seed" region of miRNAs provides most of the pairing specificity. It is believed that the first nine miRNA nucleotides (encompassing the seed sequence) provide greater specificity whereas the miRNA ribonucleotides 3' of this region allow for lower sequence specificity and thus tolerate a higher degree of mismatched base pairing, with positions 2-7 being the most important. Accordingly, the miR binding site preferably comprises a subsequence that is fully complementary (i.e., 100% complementary) over the entire length of the seed sequence of the miR.

miRNA sequences and miRNA binding sequences that can be used in the context of the present invention include, but are not limited to, all or a portion of the sequences herein discloses, as well as the miRNA precursor sequence, or complement of one or more of these miRNAs. Any aspects of the disclosure involving specific miRNAs or miRNA binding sites by name is contemplated also to cover miRNAs or complementary sequences thereof whose sequences are at least about 50%, at least about 55%, at least about 60%, at least about 65%, at least about 70%, at least about 71%, at least about 72%, at least about 73%, at least about 74%, at least about 75%, at least about 76%, at least about 77%, at least about 78%, at least about 79%, at least about 80%, at least about 81%, at least about 82%, at least about 83%, at least about 84%, at least about 85%, at least about 86%, at least about 87%, at least about 88%, at least about 89%, at least about 90%, at least about 91%, at least about 92%, at least about 93%, at least about 94%, at least about 95%, at least about 96%, at least about 97%, at least about 98%, at least about 99%, or about 100% identical to the mature sequence of the specified miRNA sequence or complementary sequence thereof.

In some embodiments, the miRNA inhibitor binds to a miRNA comprising a nucleic acid that has at least 50%, at least 51%, at least 52%, at least 53%, at least 54%, at least 55%, at least 56%, at least 57%, at least 58%, at least 59%, at least 60%, at least 61%, at least 62%, at least 63%, at least 64%, at least 65%, at least 66%, at least 67%, at least 68%, at least 69%, at least 70%, at least 71%, at least 72%, at least 73%, at least 74%, at least 75%, at least 76%, at least 77%, at least 78%, at least 79%, at least 80%, least 81%, least 82%, least 83%, least 84%, least 85%, least 86%, least 87%, least 88%, least 89%, at least 90%, at least 91%, at least 92%, at least 93%, at least 94%, at least 95%, at least 96%, at least 97%, at least 98%, at least 99% sequence identity, or at least 100% sequence identity ( e.g ., at least 99.5% sequence identity, at least 99.6% sequence identity, at least 99.7% sequence identity, at least 99.8% sequence identity, at least 99.9% sequence identity, or at least 100% sequence identity) to the nucleic acid sequence of any one of SEQ ID NOs. 17-24. In some embodiments, the miRNA inhibitor binds to a miRNA comprising a nucleic acid that is any one of SEQ ID NOs. 17-24.

In some embodiments, the miRNA inhibitors are for example at least 5, 10, 20, 25, 30, 35, 40, 45, 50, 55, 60, 65, 70, 75, 80, 85, 90, 95, 100, 105, 110, 115, or 120 nucleotides in length. In some embodiments, the miRNA inhibitors are no more than 120, 115, 110, 100, 95, 90, 85, 80, 75, 70, 65, 60, 55, 50, 45, 40, 35, 30, 25, 20, 10, or 5 nucleotides in length. In some embodiments, the miRNA inhibitors are at least 18 nucleotides in length. In some embodiments, the miRNA inhibitors are no more than 22 nucleotides in length. A miRNA inhibitor disclosed herein may comprise at least 1, 2, 3, 4, 5, 6, 7, 8, 9, 10, 11, 12, 13, 14, 15, 16, 17, 18, 19, or 20 substitutions.

A miRNA inhibitor is preferably between about 7 to 35 nucleotides (e.g., 17 to 25 nucleotides) in length and comprises a 5' to 3' sequence that is at least 90% complementary to the 5' to 3' sequence of a mature miRNA. In certain embodiments, a miRNA inhibitor molecule is 17, 18, 19, 20, 21, 22, 23, 24, or 25 nucleotides in length, or any range derivable therein. Moreover, an miRNA inhibitor has a sequence (from 5' to 3') that is or is at least 50%, at least 51%, at least 52%, at least 53%, at least 54%, at least 55%, at least 56%, at least 57%, at least 58%, at least 59%, at least 60%, at least 61%, at least 62%, at least 63%, at least 64%, at least 65%, at least 66%, at least 67%, at least 68%, at least 69%, at least 70%, at least 71%, at least 72%, at least 73%, at least 74%, at least 75%, at least 76%, at least 77%, at least 78%, at least 79%, at least 90, 91, 92, 93, 94, 95, 96, 97, 98, 99, 99.1, 99.2, 99.3, 99.4, 99.5, 99.6, 99.7, 99.8, 99.9 or 100% complementary, or any range derivable therein, to the 5' to 3' sequence of a mature miRNA, particularly a mature, naturally occurring miRNA. One of skill in the art could use that portion of the longer probe sequence that is has at least partial complementarity to at least a portion of a sequence of a mature miRNA as the sequence for a miRNA inhibitor.

In certain embodiments, a vector comprises the miRNA inhibitor as described herein.

In certain embodiments, the miRNA inhibitors or agent provided herein comprise one or more chemical modifications, wherein the modification facilitates the penetration of a cellular membrane in the absence of a delivery vehicle. Any chemical modification of the miRNA inhibitor that sustains the same functional structure will bind with its intended target. Examples of any of the chemical modifications include a 2'-O-methylated nucleoside (2OMe), a 2'-fluoro oligonucleotide (2'F), a 2'-0-methoxy ethyl oligonucleotide (2'MOE), a N6-methyladenosine (m6A), a phosphorodiamidate morpholino oligonucleotide (PMO), a peptide nucleic acid (PNA), a phosphorothioate bond (PS), a locked nucleic acid (LNA), a non-nucleotide N,N-diethyl-4-(4-nitronaphthalen-l-ylazo)-phenylamine (ZEN), a hydrophobic moiety, a naphthyl modifier, or a cholesterol moiety. In certain embodiments, the chemical modification of the miRNA is produced by methylation.

The miRNA inhibitors or agent described herein can employ a variety of oligonucleotide chemistries. Examples of oligonucleotide chemistries include, without limitation, peptide nucleic acid (PNA), locked nucleic acid (LNA), phosphorothioate, 2'0-Me-modified oligonucleotides, and morpholino chemistries, including combinations of any of the foregoing. The structures of LNAs can be found, for example, in Wengel, et al., Chemical Communications (1998) 455; Tetrahedron (1998) 54:3607, and Accounts of Chem. Research (1999) 32:301); Obika, et al., Tetrahedron Letters (1997) 38:8735; (1998) 39:5401, and Bioorganic Medicinal Chemistry (2008) 16:9230. Agents provided herein may incorporate one or more LNAs; in some cases, the compounds may be entirely composed of LNAs.

The miRNA inhibitors or agent described herein can be prepared by any appropriate method known in the art. For example, in some embodiments, the miRNA inhibitors described herein are prepared by chemical synthesis or in vitro transcription.

In the present methods, a miRNA or miRNA inhibitor or agent described herein can be administered to the subject, for example, as nucleic acid without a delivery vehicle, in combination with a delivery reagent, and/or as a nucleic acid comprising sequences that express the miRNA or the miRNA inhibitor or agent described herein. In some embodiments, any nucleic acid delivery method known in the art can be used in the methods described herein.

The microRNAs of the invention have been identified for the claimed use through a high-content, fluorescence-microscopy-based, high-throughput screen performed in primary mouse alveolar type II epithelial cells (ATII) cells using a library of 2042 miRNA mimics.

The present invention also comprises homologs, analogues and orthologues thereof, primary transcripts, precursors and mimics (also defined herein as synthetic mimics), mature miRNA molecules, and DNA molecules encoding said miRNAs or a primary transcript or a precursor or a synthetic mimic or a functional derivative thereof or the above agent, for the uses herein disclosed for the present miRNAs and inhibitors and agents. The concepts of microRNA primary transcript, precursor and mimic are well-known in the art and do not require further explanations. In particular, for microRNA mimic it is intended a RNA molecule intended to "mimic" the activity and function of the native microRNA. The use of nucleic acid or DNA coding for the agent or inhibitor or miRNA or primary transcript or precursor or synthetic mimic or functional derivative thereof as defined above is also within the scope of the present invention. Such DNA, for example a cDNA, can be designed according to the general knowledge in the field.

As defined herein, the term "functional derivative" of a miRNA refers to a miRNA that has less than 100% identity to a corresponding wild-type miRNA and possesses one or more biological activities of the corresponding wild-type miRNA. Examples of such biological activities include, but are not limited to, inhibition of expression of a target RNA molecule (e.g., inhibiting translation of a target mRNA molecule and/or modulating the stability of a target mRNA molecule) and inhibition of a cellular process associated therewith. These functional derivatives include species variants and variants that are the consequence of one or more mutations (e.g., a substitution, a deletion, an insertion) in a miRNA-encoding gene. In certain embodiments, the variant is at least about 87%, 90%, 95%, 98%, or 99% identical to a corresponding wild-type miRNA. Functional derivatives also encompass "functional fragments" of a miRNA, i.e., portions of miRNA which are less than the full-length molecule (and their species and mutant variants) and that possess one or more biological activities of a corresponding wild-type miRNA. In certain embodiments, the biologically-active fragment is at least about 7, 10, 12, 15, or 17 nucleotides in length. In other embodiments, the biologically active fragment is at least 7 or more nucleotides, preferably at least 8 or more nucleotides. The functional derivative may also include longer sequences or shifted sequences, compared to the miRNA; optionally, the functional derivative may include longer sequences or shifted sequences from said miRNA genomic sequence.

Also included in the present invention are variants of primary miRNA and of precursor miRNAs of the invention comprising a sequence with at least 50%, 60%, 70%, 75%, 80%, 85%, 90%, 95% identity to the reference sequences or DNA molecules encoding said miRNAs.

It has been found that microRNAs hsa-miR-124-3p, hsa-miR-5008-5p, hsa-miR-4255, hsa-miR-4691-5p, hsa-miR-4781-5p, hsa-miR-4782-5p, hsa-miR-3928-3p scored as the most effective in promoting trans-differentiation of ATII cells into ATI cells with a Z-Score (a statistic parameter showing how many standard deviations a measurement is far from the mean of all treatments) higher than 1.96 (p≤0.05) at 3 days post isolation.

It has been found that hsa-miR-183-3p, hsa-miR-16-2-3p, hsa-miR-147b-3p, hsa-miR-1292-3p scored as the most effective in blocking trans-differentiation of ATII cells into ATI cells with a Z-Score (a statistic parameter showing how many standard deviations a measurement is far from the mean of all treatments) lower than 1.96 (p≤0.05) at 3 days post isolation

Both Untreated ATII cells and non-targeting microRNA-4 (MC4, cel-miR-231-3p) treated ATII cells scored a non-significant Z-Score (Z≤1,96).

Therefore, the above microRNAs are able to effectively either promote (microRNAs hsa-miR-124-3p, hsa-miR-5008-5p, hsa-miR-4255, hsa-miR-4691-5p, hsa-miR-4781-5p, hsa-miR-4782-5p, hsa-miR-3928-3p) or inhibit (microRNAs hsa-miR-183-3p, hsa-miR-16-2-3p, hsa-miR-147b-3p, hsa-miR-1292-3p) the trans-differentiation of primary murine ATII cells into ATI cells.

Therefore, said microRNAs and inhibitors can be used for the treatment of all lung diseases in which the loss of either ATII or ATI cells is the driver of the disease (for example, ILDs, IPF, chronic obstructive pulmonary (or lung) disease, acute respiratory distress syndrome). The present agents can therefore be used for medical use, in particular for the treatment of pathological conditions associated with loss of alveolar epithelial type II and/or type I cells and/or with loss of lung regenerative capacity and/or for use in promoting lung regeneration, wherein the pathological condition is preferably selected from the group of interstitial lung diseases, preferably idiopathic pulmonary fibrosis, non-specific interstitial pneumonia, desquamative interstitial pneumonia, respiratory bronchiolitis-ILD, cryptogenic organizing pneumonia, acute interstitial pneumonia, chronic obstructive pulmonary diseases, acute and post-acute respiratory distress syndrome, post-acute COVID-19 syndrome.

The present agents can therefore be used for the treatment of interstitial lung diseases, preferably idiopathic pulmonary fibrosis, non-specific interstitial pneumonia, desquamative interstitial pneumonia, respiratory bronchiolitis-ILD, cryptogenic organizing pneumonia, acute interstitial pneumonia, chronic obstructive pulmonary diseases, acute and post-acute respiratory distress syndrome, post-acute COVID-19 syndrome.

The skilled person in the field, for example a physician specialized in the lung disease, is able to identify the pathologies characterized by pathological remodelling of lung architecture, fibrosis, loss of alveoli, and eventually reduced gas-exchange capacity, according to the common knowledge in the field, which may characterize the above indicated conditions.

Said microRNA or agents can also be advantageously used in any condition at risk of developing lung diseases as defined above.

Indeed, for prevention it is intended the administration of the microRNA or agent to a subject liable or at risk to develop a condition herein mentioned due, for example, to genetic predisposition (e.g. familial Pulmonary fibrosis), environment conditions (e.g. organic dust (livestock/agriculture/farming), metal and mineral dust, wood dust, asbestos, cigarette smoking) or the presence of some conditions or pathologies eventually leading to any pathological conditions associated with loss of alveolar epithelial type II and/or type I cells and/or with loss of lung regenerative capacity and/or for use in promoting lung regeneration, wherein the pathological condition is preferably selected from the group of interstitial lung diseases, preferably idiopathic pulmonary fibrosis, non-specific interstitial pneumonia, desquamative interstitial pneumonia, respiratory bronchiolitis-ILD, cryptogenic organizing pneumonia, acute interstitial pneumonia, chronic obstructive pulmonary diseases, acute and post-acute respiratory distress syndrome, post-acute COVID-19 syndrome.

The use of the present agents in gene therapy is also a preferred embodiment of the invention.

For gene therapy it is intended the therapeutic delivery of nucleic acids into a patient's cells as a drug to treat a disease. According to the present invention, the agent of the invention can be delivered to cells of a subject in need thereof, for example a subject affected by or at risk of developing any of the above mentioned diseases, in order to treat such diseases.

A further obj ect of the present invention is an RNA stretch comprising the said microRNA or inhibitor. The notion of RNA stretch as a continuous tract of RNA is commonly known in the field. Said RNA stretch can be obtained in vitro through cell-free transcription methods, produced synthetically or expressed in the cells upon transfer of the relative DNA coding sequence, or introduced or expressed in the cells by administration of a plasmid, a viral or other type of vector.

The present invention provides said RNA stretch for use as a medicament for prevention and/or treatment of any of the above mentioned diseases.

In embodiments of the present invention, the agent of the invention can be administered to a subject as a medicament by conventional methods.

Conveniently, said medicament or agent or pharmaceutical composition as defined herein is in the form of a preparation for intra-tracheal instillation, inhalation (Aerosol), parenteral administration, intravenous administration, but other forms are equally suitable for carrying out the present invention. The person skilled in the art will decide the effective time of administration, depending on the patient's conditions, degree of severity of the disease, response of the patient and any other clinical parameter within the general knowledge of this matter.

Another object of the present invention is a pharmaceutical composition comprising the above defined agent as active ingredient for the prevention and/or treatment of any of the above mentioned diseases.

The pharmaceutical composition according to the invention may contain at least one of the following: a microRNA, synthetic RNA corresponding to the microRNA of the present invention or its primary transcript or precursor or synthetic mimic or functional derivative thereof, DNA coding for said microRNA or its primary transcript or precursor or synthetic mimic or functional derivative thereof, DNA coding for a primary transcript or precursor for said RNA such as the microRNA is produced inside the cells containing this DNA.

The agent or microRNA (or primary transcript or precursor or synthetic mimic or functional derivative thereof) of the present invention or the corresponding coding DNA can be administered naked or together with lipid nanoparticle or lipidic molecules such as cationic lipids, or peptides, or in the context of polymeric scaffolds, which can facilitate their delivery, according to the art.

When the agent is administered using as a vector a lipidic molecule or nanoparticle, such as a liposome, the sequence of the miRNA is preferably SEQ ID NO: 4, 6, 8, 10, 12,14, 16, 18, 20, 22 or 24 (corresponding to mature miRNA sequences).

Another method to administer such microRNA or agent or its corresponding DNA is by means of a suitable vector known for the administration of RNA or DNA.

A preferred vector is the adeno-associated vector (AAV) of any capsid serotype, either natural (such as, but not restricted to, AAV6, AAV1, AAV2, AAV8 or AAV9) or artificial (AAV6.2, AAV6.2FF) [36] [37].

When the agent is administered using as a vector a viral vector, the sequence of the miRNA is preferably SEQ ID NO: 1, 2, 3, 5, 7, 9, 11, 13, 15, 17, 19, 21 or 23 (corresponding to stem-loop sequences).

A vector for use according to the present invention comprising at least the agent as defined above and/or a DNA coding for at least said agent and/or a DNA coding for at least a primary transcript or a precursor or a mimic of said agent, or a combination thereof is also an object of the invention.

All these methods and formulations to administer a microRNA or synthetic RNA or synthetic mimic corresponding to the agent of the present invention, DNA coding for said agent, DNA coding for a primary transcript or precursor for microRNA such as the microRNA is produced inside the cells containing this DNA, are conventional and well known in the art and do not need further explanations. In particular, the skilled person knows how to choose the suitable administration mode and vector, for example for human administration, according to the general knowledge in the field.

Injection is a preferred administration route. However, the skilled person in the art can decide to administer the agents by means of any conventional pharmaceutical composition. Reference can be made to Remington's Pharmaceutical Sciences, last edition.

The administration regime, dosage and posology will be determined by the physician according to his experience, the disease to be treated and the patient's conditions.

According to the administration route chosen, the compositions will be in solid, liquid or nebulized form, suitable for oral, parenteral, intravenous or intra-tracheal administration.

The compositions according to the present invention contain, along with the agent, at least one pharmaceutically acceptable vehicle or excipient. These may be particularly useful formulation co-adjuvants, e.g. solubilising agents, dispersing agents, suspension agents, and emulsifying agents. Average quantities of the active agent may vary and in particular should be based upon the recommendations and prescription of a qualified physician.

The present invention also refers to the herein defined agents, pharmaceutical compositions, nucleic acids, vectors, host cells, recombinant adeno-associated virus (rAAV) particles and also refers to their medical use.

The present invention also refers to the herein defined agents, pharmaceutical compositions, nucleic acids, vectors, host cells, recombinant adeno-associated virus (rAAV) particles for use in the prevention and/or treatment of an interstitial lung disease.

The present invention also refers to the herein defined agents, pharmaceutical compositions, nucleic acids, vectors, host cells, recombinant adeno-associated virus (rAAV) particles for use in the prevention and/or treatment of idiopathic pulmonary fibrosis, non-specific interstitial pneumonia, desquamative interstitial pneumonia, respiratory bronchiolitis-ILD, cryptogenic organizing pneumonia, acute interstitial pneumonia, chronic obstructive pulmonary diseases or acute and post-acute respiratory distress syndrome or post-acute COVID-19 syndrome.

In the context of the present invention, the lung pathological condition associated with loss of alveolar epithelial type II and/or type I cells and/or with loss of lung regenerative capacity may be an interstitial lung disease.

In the context of the present invention, the lung pathological condition associated with loss of alveolar epithelial type II and/or type I cells and/or with loss of lung regenerative capacity may be idiopathic pulmonary fibrosis, non-specific interstitial pneumonia, desquamative interstitial pneumonia, respiratory bronchiolitis-ILD, cryptogenic organizing pneumonia, acute interstitial pneumonia, chronic obstructive pulmonary diseases or acute and post-acute respiratory distress syndrome or post-acute COVID-19 syndrome.

In the context of the present invention, the lung pathological condition associated with loss of alveolar epithelial type II and/or type I cells and/or with loss of lung regenerative capacity may be idiopathic pulmonary fibrosis, chronic obstructive pulmonary diseases or acute and post-acute respiratory distress syndrome.

In the context of the present invention, the lung condition may be an interstitial lung disease.

In the context of the present invention, the lung condition may be idiopathic pulmonary fibrosis, non-specific interstitial pneumonia, desquamative interstitial pneumonia, respiratory bronchiolitis-ILD, cryptogenic organizing pneumonia, acute interstitial pneumonia, chronic obstructive pulmonary diseases or acute and post-acute respiratory distress syndrome or post-acute COVID-19 syndrome. In the context of the present invention, the lung condition may be idiopathic pulmonary fibrosis, chronic obstructive pulmonary diseases or acute and post-acute respiratory distress syndrome.

In the context of the present invention, the interstitial lung disease is preferably idiopathic pulmonary fibrosis, non-specific interstitial pneumonia, desquamative interstitial pneumonia, respiratory bronchiolitis-ILD, cryptogenic organizing pneumonia or acute interstitial pneumonia.

It is also an object of the present invention a method of prevention and/or treatment of lung pathological conditions associated with loss of alveolar epithelial type II and/or type I cells and/or with loss of lung regenerative capacity, comprising administering at least one agent, pharmaceutical composition, nucleic acid, vector, host cell, recombinant adeno-associated virus (rAAV) particle or combination thereof as defined herein to a patient in need thereof.

It is also an object of the present invention a method of promoting lung regeneration in a subject with a lung condition, comprising administering at least one agent, pharmaceutical composition, nucleic acid, vector, host cell, recombinant adeno-associated virus (rAAV) particle or combination thereof as defined herein to a patient in need thereof.

The present invention will now be illustrated in reference to the following figures and examples.

### Brief description of the figures

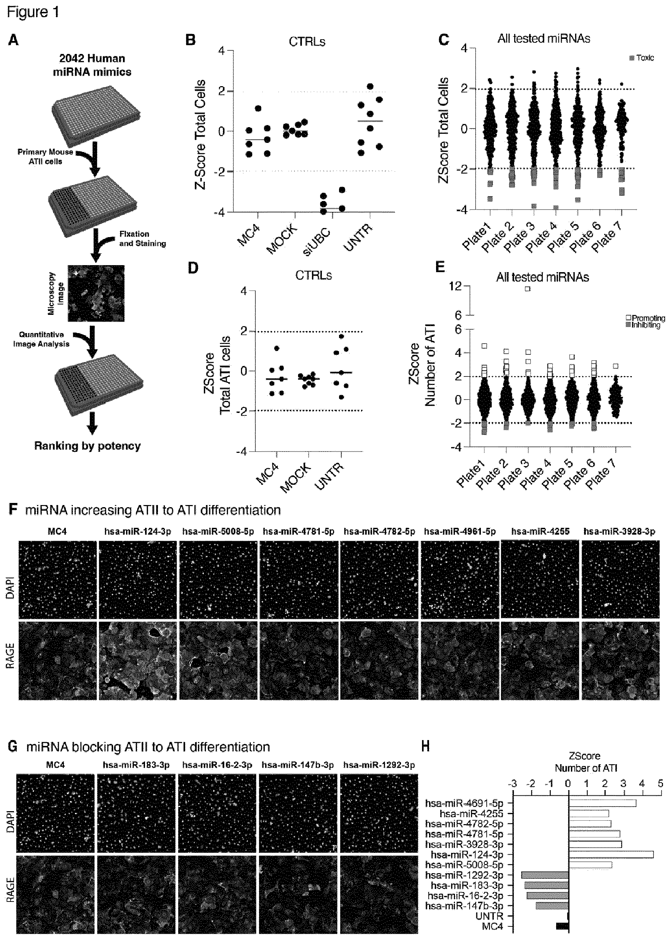

Figure 1. Human microRNA screening in primary mouse ATII cells for ATII to ATI trans-differentiation. A. Screening workflow. Briefly 2042 human microRNA mimics were reverse-transfected (final concentration 25nM) into primary mouse ATII cells and at 72 hours after transfection cell were fixed in PFA and immune-stained for RAGE. Plates were imaged and images were analyzed by counting the total cell number and the total number of RAGE-positive cells. All non-toxic miRNAs were ranked for potency in either promoting or blocking ATII to ATI trans-differentiation. B. Chart showing Z-Score of total cell number in CTRL conditions, cells treated with Optimem and RNAiMAX (MOCK), cells treated with negative control microRNA (MC4), untreated cells (UNTR), cell treated with siUBC, positive transfection control (siUBC). C. Chart showing Z-Score of the total cell number in all screening conditions, microRNAs corresponding to squared gray dots are considered significantly toxic (Z≤-1,96, P≤0,05). D. Chart showing Z-Score of the total number of ATI cells in control conditions. E. Chart showing Z-Score of the total number of ATI cells in all screening conditions. In particular, microRNAs corresponding to squared white dots are considered significantly increasing the number of ATI cells/well (Z≥1,96, P≤0,05); microRNAs corresponding to squared grey dots are considered significantly decreasing the number of ATI cells/well (Z≤-1,96, P≤0,05). F. Representative immunofluorescence images of miRNAs increasing ATII to ATI trans-differentiation. G. Representative immunofluorescence images of miRNAs decreasing ATII to ATI trans-differentiation. H. Quantification of images shown in F and G, Z-Score of the total number of ATI cells/well for selected miRNAs and control are plotted.

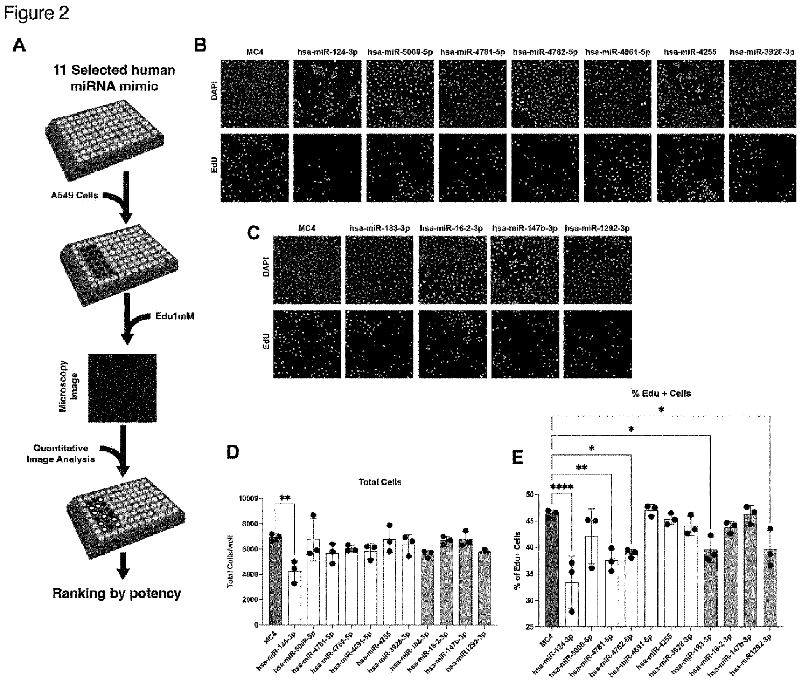

Figure 2. Effect of 11 selected Human microRNAs on A549 adenocarcinoma cell line. A. Experimental scheme. Briefly 11 human microRNA mimics were reverse-transfected (final concentration 25nM) into A549 cells and at 70 hours after transfection cells were pulsed with 1mM EdU. At 72 hours after transfection cell were fixed in PFA and stained for EdU incorporation. Plates were imaged and images were analyzed by counting the total cell number and the total number of EdU-positive cells. MicroRNAs were ranked for potency in either promoting or blocking A549 EdU incorporation. B. Representative images of A549 cells treated with miRNAs increasing ATII to ATI trans-differentiation and stained for EdU incorporation. C. Representative immunofluorescence images of A549 cells treated with miRNAs decreasing ATII to ATI trans-differentiation and stained for EdU incorporation. D. Quantification of total cell number of A549 cells/well. E. Quantification of the % of EdU positive cells over total number of cells. Data from 3 independent experiments/conditions were analyzed by One-Way ANOVA followed by Dunnet correction for multiple comparisons and statistical significance is reported as it follows: ^{∗∗∗∗}P≤0.0001, ^{∗∗} P≤0,005, ^{∗}P≤0.05.

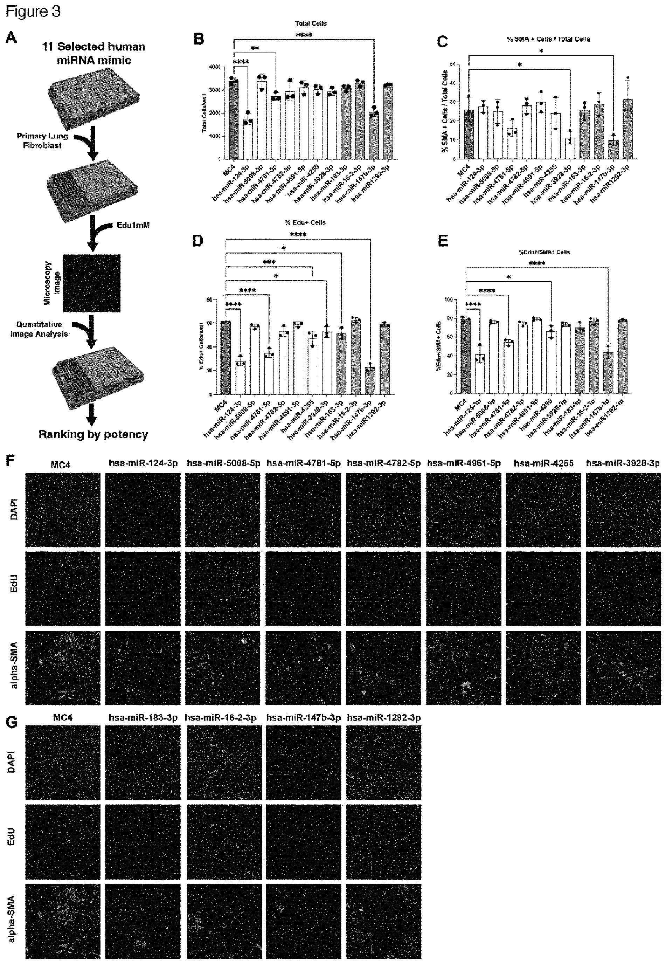

Figure 3. Effect of 11 selected Human microRNAs on primary lung alveolar fibroblasts. A. Experimental scheme. Briefly 11 human microRNA mimics were reverse-transfected (final concentration 12.5nM) into primary lung alveolar fibroblasts and at 42 hours after transfection cells were pulsed with 1mM EdU. At 48 hours after transfection cell were fixed in PFA and stained for EdU incorporation and alpha-SMA positivity. Plates were imaged and images were analyzed by counting the total cells number, the total number of EdU-positive cells, and the total number of alpha-SMA-positive cells. MicroRNAs were ranked for potency in either promoting or blocking EdU incorporation and alpha-SMA positivity. Effect of the 11 selected microRNAs on (B) quantification of the total cell number/well, (C) quantification of the % of alpha-SMA positive cells over total cells/well, (D) quantification of the % of EdU positive cells over total cells/well, (E) quantification of the % of EdU positive cells over alpha-SMA positive cells /well. F. Representative images of primary lung alveolar fibroblasts treated with miRNAs increasing ATII to ATI trans-differentiation and stained for EdU and alpha-SMA positivity. G. Representative images of primary lung alveolar fibroblasts treated with miRNAs decreasing ATII to ATI trans-differentiation and stained for EdU and alpha-SMA positivity. Data from 3 independent experiments/conditions were analyzed by One-Way ANOVA followed by Dunnet correction for multiple comparisons and statistical significance is reported as it follows: ^{∗∗∗∗}P≤0.0001; ^{∗∗∗} P≤0.0005; ^{∗∗} P≤0,005; ^{∗}P≤0.05.

### EXAMPLES

### Primary Screening Results

Inventors performed a cell-based phenotypic high throughput screening (HTS) assay on 2042 human microRNA mimics for their efficacy in promoting ATII to ATI trans-differentiation in primary alveolar mouse epithelial cells and they identified 47 miRNA mimics that significantly (P≤0.05) increased ATII to ATI trans-differentiation and 20 miRNAs that significantly decreased ATII to ATI trans-differentiation. Inventors developed a protocol for the purification and culture of mouse alveolar ATII cells (>88% purity). After isolation, cells were reverse-transfected with an arrayed library of 2042 human miRNA mimics at 25nM final concentration (Horizon Discovery) (Experimental scheme in Figure 1A). In particular, 5µl of miRNA mimic (250nM) were spotted by a Hamilton Starlet Liquid Handler on the bottom of 384-wells-phenoplate (PerkinElmer #6057300) previously coated in fibronectin gelatin. Immediately after a mixture composed of 15µl of Optimem (Gibco) and 0.15 lipofectamine RNAimax was added to each well, mixed and incubated for 30 minutes at room temperature. After effective incubation, 15.000 cells diluted in a final volume of 30µl were added to each well. The assay was stopped at 72 hours post transfection and cells were stained with fluorescent probes for the following markers: 1) Rage (ATI cells) and Hoechst 3342 (nuclei). An average of 1000 cells/condition have been imaged by automated fluorescence microscopy (Operetta CLS microscope) and a complete phenotypic profiling has been performed for all treatments.

ATII cells were effectively transfected as shown in Figure 1B, where the positive control siUBC shown effective cell killing upon transfection; on the contrary the negative control non-targeting microRNA-4 (MC4, cel-miR-231-3p) didn't show any toxicity scoring a non-significant Z-Score (Z≤1,96). Both Untreated, Mock transfected (only RNAimax and Optimem), and MC4 treated ATII cells, once cultured, spontaneously trans-differentiate into ATI cells with a Z-score of 0.142 (Untreated condition) at 3 days post isolation (Figure 1D).

Inventors excluded toxic miRNAs significantly reducing the number of cells/well (Z-Score viability < -1.96, P≤0.05, Squared dot in Figure1C). According to these criteria, we identified 47 miRNA mimics that significantly (P≤0.05) increased ATII to ATI trans-differentiation and 20 miRNAs that significantly decreased ATII to ATI trans-differentiation (respectively Z-Score number of ATI cells ≥ 1.96, P≤0.05; and Z-Score number of ATI cells ≤ -1.96, P≤0.05; Squared dot in Figure1E). Inventors then proceeded with image inspection of all selected miRNAs and they confirmed 7 human miRNAs as the most effective in promoting ATII to ATI trans-differentiation and 4 human miRNAs as the most effective in blocking ATII to ATI trans-differentiation (Representative IF images in Figure 1F and G and **Table 1**).

**Table 1**

| ***miRNA*** | ***miRbase ID*** | ***Mature sequence 5'-3'*** | ***miRbase ID*** | ***Pre-miRNA sequence 5'-3'*** |
|---|---|---|---|---|
| ***hsa-miR-124-3p*** | *MIMAT00004* 22 | UAAGGCACGCG GUGAAUGCCAA SEQ ID NO: 4 | MI000044 3 | AGGCCUCUCUCUCCGUGUU CACAGCGGACCUUGAUUUAA AUGUCCAUACAAUUAAGGCA CGCGGUGAAUGCCAAGAAUG GGGCUG SEQ ID NO: 1 |
| | | | MI000044 4 | AUCAAGAUUAGAGGCUCUGC UCUCCGUGUUCACAGCGGAC CUUGAUUUAAUGUCAUACAA UUAAGGCACGCGGUGAAUGC CAAGAGCGGAGCCUACGGCU GCACUUGAA SEQ ID NO: 2 |
| | | | MI000044 5 | UGAGGGCCCCUCUGCGUGU UCACAGCGGACCUUGAUUUA AUGUCUAUACAAUUAAGGCA CGCGGUGAAUGCCAAGAGAG GCGCCUCC SEQ ID NO: 3 |
| ***hsa-miR-5008-5p*** | *MIMAT00210* 39 | UGAGGCCCUUG GGGCACAGUGG SEQ ID NO: 6 | MI001787 6 | GGGCUGACCCCUAGGGUCA GGUGAGGCCCUUGGGGCAC AGUGGUGCCAUCUCCCCUG UGCUCCCAGGGCCUCGCCU GUCCCUUGAGGUCGGCCC SEQ ID NO: 5 |
| **hsa-miR-4255** | *MIMAT00168 85* | CAGUGUUCAGA GAUGGA SEQ ID NO: 8 | MI001586 3 | GAGCAUCCUUCAGUGUUCA GAGAUGGAGUCAGUAUUGG UCUGGCCAUUUUUAGGGCA AAGAGGCAGCAUCAU SEQ ID NO: 7 |
| ***hsa-miR-4691-5p*** | *MIMAT00197 81* | GUCCUCCAGGC CAUGAGCUGCG G SEQ ID NO: 10 | MI001732 4 | GGAGCACUCCCAGGUCCUC CAGGCCAUGAGCUGCGGCC CUGAUGUCUCUACUCCAGC CACGGACUGAGAGUGCAUA GGAGUGUCC SEQ ID NO: 9 |
| **hsa-miR-4781-5p** | *MIMAT00199* 42 | UAGCGGGAUU CCAAUAUUGG SEQ ID NO: 12 | MI001742 6 | AGGUGCACGCUCUAGCGGG GAUUCCAAUAUUGGGCCAA UUCCCCCAAUGUUGGAAUC CUCGCUAGAGCGUGCACUU SEQ ID NO: 11 |
| **hsa-miR-4782-5p** | *MIMAT00199* 44 | UUCUGGAUAUG AAGACAAUCAA SEQ ID NO: 14 | MI001742 7 | AUUGCCCAGUUCUGGAUAU GAAGACAAUCAAGAAAAGA UUUGGUGUUCUUGAUUGUC |
| | | | | UUCAUAUCUAGAACUGGGC AGU SEQ ID NO: 13 |
| hsa-miR-3928-3p | MIMAT00182 05 | GGAGGAACCUU GGAGCUUCGGC SEQ ID NO: 16 | MI001643 8 | GCUGAAGCUCUAAGGUUCC GCCUGCGGGCAGGAAGCGG AGGAACCUUGGAGCUUCGG C SEQ ID NO: 15 |
| hsa-miR-183-3p | MIMAT00045 60 | GUGAAUUACCG AAGGGCCAUAA SEQ ID NO: 18 | MI000027 3 | CCGCAGAGUGUGACUCCUG UUCUGUGUAUGGCACUGGU AGAAUUCACUGUGAACAGU CUCAGUCAGUGAAUUACCG AAGGGCCAUAAACAGAGCA GAGACAGAUCCACGA SEQ ID NO: 17 |
| hsa-miR-16-2-3p | MIMAT00045 18 | CCAAUAUUACU GUGCUGCUUUA SEQ ID NO: 20 | MI000011 5 | GUUCCACUCUAGCAGCACG UAAAUAUUGGCGUAGUGAA AUAUAUAUUAAACACCAAU AUUACUGUGCUGCUUUAGU GUGAC SEQ ID NO: 19 |
| hsa-mir-147b-3p | MIMAT00049 28 | GUGUGCGGAAA UGCUUCUGCU SEQ ID NO: 22 | MI000554 4 | UAUAAAUCUAGUGGAAACA UUUCUGCACAAACUAGAUU CUGGACACCAGUGUGCGGA AAUGCUUCUGCUACAUUUU UAGG SEQ ID NO: 21 |
| hsa-miR-1292-3p | MIMAT00229 48 | UCGCGCCCGG CUCCCGUUC SEQ ID NO: 24 | MI000643 3 | CCUGGGAACGGGUUCCGGC AGACGCUGAGGUUGCGUUG ACGCUCGCGCCCCGGCUCCC GUUCCAGG SEQ ID NO: 23 |

### Effect on Adenoma lung cancer cell line

Next, to evaluate the eventual oncogenic effect of the 11 selected miRNAs, inventors reverse-transfected adenocarcinoma cell line (A549 cells) with the selected 11 human miRNA mimics at 25nM final concentration (Horizon Discovery). In particular, 7,5µl of miRNA mimic (250nM) were spotted on the bottom of 96-wells-phenoplate (PerkinElmer #6057300). Immediately after a mixture composed of 41,95µl of Optimem (Gibco) and 0.4 µl lipofectamine RNAimax was added to each well, mixed and incubated for 30 minutes at room temperature. After effective incubation, 3.000 cells diluted in a final volume of 100µl were added to each well. The assay was stopped at 72 hours post transfection. To investigate whether selected miRNAs also resulted in modulating cancer-cell proliferation, inventors added EdU 2 hours before fixing (Figure 2A). Finally, cells were stained with fluorescent probe for EdU (Proliferation) and Hoechst 3342 (nuclei). An average of 1500 cells/condition have been imaged by automated fluorescence microscopy (Operetta CLS microscope) and a complete profiling has been performed for all treatments. In particular, inventors can observe that 1) hsa-miR-124-3p significantly decreased both the total cells number and the % of EdU+ cells; 2) hsa-miR-4781-5p, hsa-miR-4782-5p, hsa-miR-183-3p and, hsa-miR-1292-3p significantly decreased % of EdU+ cells (representative IF images in figure 2B, C and quantifications in figure 2D, E). These results together indicate that none of the selected miRNAs have a proliferative effect on lung tumour cell lines.

### Effect on primary Alveolar Lung Fibroblasts

Finally, to further investigate eventual effect of the selected miRNAs to other cell type in the alveolus, inventors isolated primary alveolar lung fibroblasts from the distal portion of lungs of adult C57BL/6 mice. After the isolation, they reverse-transfected primary alveolar lung fibroblasts with the 6 human miRNA mimics at 12,5nM final concentration (Horizon Discovery). In particular, 3,75µl of miRNA mimic (250nM) were spotted on the bottom of 96-wells-phenoplate (PerkinElmer #6057300). Immediately after a mixture composed of 45,85µl of Optimem (Gibco) and 0.4 µl lipofectamine RNAimax was added to each well, mixed and incubated for 30 minutes at room temperature. After effective incubation, 4.000 cells diluted in a final volume of 100µl were added to each well. The assay was stopped at 48 hours post transfection and EdU was added onto the medium 6h before fixing (experimental scheme in Figure 3A). Finally, cells were stained with fluorescent probe for α-SMA (activated fibroblasts), EdU (Proliferation) and Hoechst 3342 (nuclei). An average of 1500 cells/condition have been imaged by automated fluorescence microscopy (Operetta CLS microscope) and a complete phenotypic profiling has been performed for all treatments. We found that all selected miRNAs are not promoting neither fibroblast proliferation nor increasing the number of alpha-SMA positive cells, both markers of pathological fibroblast to myofibroblast activation. In addition, 1) hsa-miR-124-3p transfection decreases the number of total cells, the % of EdU+ cells and the % of Edu+/alpha-SMA+ cells, suggesting a role in blocking the transition of fibroblasts into myofibroblasts; 2) hsa-miR-4781 and hsa-miR-147b-3p transfection decreases the number of total cells, the % of EdU+ cells, the % of alpha-SMA+ cells, and the % of Edu+/alpha SMA+ cells, suggesting a role in blocking the transition of fibroblasts into myofibroblasts (quantifications in figure 3B,C,D, E and representative IF images in figure 3F, G). These results together indicate that none of the selected miRNAs induces a pro-fibrotic profile and some of the selected miRNAs showed an anti-fibrotic effect on primary alveolar fibroblasts *in vitro,* thus further supporting their potential efficacy in treating respiratory disease characterized by progressive fibrotic scarring of the lung.

### BIBLIOGRAPHY

1. Ley, B. and H.R. Collard, Epidemiology of idiopathic pulmonary fibrosis. Clin Epidemiol, 2013. 5: p. 483-92.
2. Rock, J.R., et al., Multiple stromal populations contribute to pulmonary fibrosis without evidence for epithelial to mesenchymal transition. Proc Natl Acad Sci USA, 2011. 108(52): p. E1475-83.
3. Barkauskas, C.E., et al., Type 2 alveolar cells are stem cells in adult lung. J Clin Invest, 2013. 123(7): p. 3025-36.
4. Desai, T.J., D.G. Brownfield, and M.A. Krasnow, Alveolar progenitor and stem cells in lung development, renewal and cancer. Nature, 2014. 507(7491): p. 190-4.
5. Adams, T.S., et al., Single-cell RNA-seq reveals ectopic and aberrant lung-resident cell populations in idiopathic pulmonary fibrosis. Sci Adv, 2020. 6(28): p. eaba1983.
6. Choi, J., et al., Inflammatory Signals Induce AT2 Cell-Derived Damage-Associated Transient Progenitors that Mediate Alveolar Regeneration. Cell Stem Cell, 2020. 27(3): p. 366-382 e7.
7. Habermann, A.C., et al., Single-cell RNA sequencing reveals profibrotic roles of distinct epithelial and mesenchymal lineages in pulmonary fibrosis. Sci Adv, 2020. 6(28): p. eaba1972.
8. Kobayashi, Y., et al., Persistence of a regeneration-associated, transitional alveolar epithelial cell state in pulmonary fibrosis. Nat Cell Biol, 2020. 22(8): p. 934-946.
9. Strunz, M., et al., Alveolar regeneration through a Krt8+ transitional stem cell state that persists in human lung fibrosis. Nat Commun, 2020. 11(1): p. 3559.
10. Basil, M.C., et al., Human distal airways contain a multipotent secretory cell that can regenerate alveoli. Nature, 2022. 604(7904): p. 120-126.
11. Eulalio, A., E. Huntzinger, and E. Izaurralde, Getting to the root of miRNA-mediated gene silencing. Cell, 2008. 132(1): p. 9-14.
12. Filipowicz, W., S.N. Bhattacharyya, and N. Sonenberg, Mechanisms of post-transcriptional regulation by microRNAs: are the answers in sight? Nat Rev Genet, 2008. 9(2): p. 102-14.
13. Ghildiyal, M. and P.D. Zamore, Small silencing RNAs: an expanding universe. Nat Rev Genet, 2009. 10(2): p. 94-108.
14. Boateng, E. and S. Krauss-Etschmann, miRNAs in Lung Development and Diseases. Int J Mol Sci, 2020. 21(8).
15. Harris, K.S., et al., Dicer function is essential for lung epithelium morphogenesis. Proc Natl Acad Sci USA, 2006. 103(7): p. 2208-13.
16. Stolzenburg, L.R. and A. Harris, The role of microRNAs in chronic respiratory disease: recent insights. Biol Chem, 2018. 399(3): p. 219-234.
17. Montgomery, R.L., et al., MicroRNA mimicry blocks pulmonary fibrosis. EMBO Mol Med, 2014. 6(10): p. 1347-56.
18. Pandit, K.V., J. Milosevic, and N. Kaminski, MicroRNAs in idiopathic pulmonary fibrosis. Trans! Res, 2011. 157(4): p. 191-9.
19. Liu, Y., et al., MiRNA, a New Treatment Strategy for Pulmonary Fibrosis. Curr Drug Targets, 2021. 22(7): p. 793-802.
20. Miao, C., et al., MicroRNAs in idiopathic pulmonary fibrosis, new research progress and their pathophysiological implication. Exp Lung Res, 2018. 44(3): p. 178-190.
21. Yang, S., et al., Participation of miR-200 in pulmonary fibrosis. Am J Pathol, 2012. 180(2): p. 484-93.
22. Katsuno, Y. and R. Derynck, Epithelial plasticity, epithelial-mesenchymal transition, and the TGF-beta family. Dev Cell, 2021. 56(6): p. 726-746.
23. Pandit, K.V., et al., Inhibition and role of let-7d in idiopathic pulmonary fibrosis. Am J Respir Crit Care Med, 2010. 182(2): p. 220-9.
24. Mansour, S.M., H.S. El-Abhar, and A.A. Soubh, MiR-200a inversely correlates with Hedgehog and TGF-beta canonical/non-canonical trajectories to orchestrate the anti-fibrotic effect of Tadalafil in a bleomycin-induced pulmonary fibrosis model. Inflammopharmacology, 2021. 29(1): p. 167-182.
25. Moimas, S., et al., miR-200 family members reduce senescence and restore idiopathic pulmonary fibrosis type II alveolar epithelial cell transdifferentiation. ERJ Open Res, 2019. **5**(4).
26. Huang, C., Y. Yang, and L. Liu, Interaction of long noncoding RNAs and microRNAs in the pathogenesis of idiopathic pulmonary fibrosis. Physiol Genomics, 2015. 47(10): p. 463-9.
27. Marts, L.T., et al., MiR-21-Mediated Suppression of Smad7 Induces TGFbeta1 and Can Be Inhibited by Activation of Nrf2 in Alcohol-Treated Lung Fibroblasts. Alcohol Clin Exp Res, 2017. 41(11): p. 1875-1885.
28. Cushing, L., P. Kuang, and J. Lu, The role of miR-29 in pulmonary fibrosis. Biochem Cell Biol, 2015. 93(2): p. 109-18.
29. Liang, C., et al., The anti-fibrotic effects of microRNA-153 by targeting TGFBR-2 in pulmonary fibrosis. Exp Mol Pathol, 2015. 99(2): p. 279-85.
30. Baumann, V. and J. Winkler, miRNA-based therapies: strategies and delivery platforms for oligonucleotide and non-oligonucleotide agents. Future Med Chem, 2014. 6(17): p. 1967-84.
31. Szczepanek, J., M. Skorupa, and A. Tretyn, MicroRNA as a Potential Therapeutic Molecule in Cancer. Cells, 2022. 11(6).
32. Dasgupta, I. and A. Chatterjee, Recent Advances in miRNA Delivery Systems. Methods Protoc, 2021. 4(1).
33. Zununi Vahed, S., et al., Liposome-based drug co-delivery systems in cancer cells. Mater Sci Eng C Mater Biol Appl, 2017. 71: p. 1327-1341.
34. O'Neill, C.P. and R.M. Dwyer, Nanoparticle-Based Delivery of Tumor Suppressor microRNA for Cancer Therapy. Cells, 2020. 9(2).
35. Herrera-Carrillo, E., Y.P. Liu, and B. Berkhout, Improving miRNA Delivery by Optimizing miRNA Expression Cassettes in Diverse Virus Vectors. Hum Gene Ther Methods, 2017. 28(4): p. 177-190.
36. van Lieshout, L.P., et al., A Novel Triple-Mutant AAV6 Capsid Induces Rapid and Potent Transgene Expression in the Muscle and Respiratory Tract of Mice. Mol Ther Methods Clin Dev, 2018. 9: p. 323-329.
37. Rindler, T.N., et al., Efficient Transduction of Alveolar Type 2 Cells with Adeno-associated Virus for the Study of Lung Regeneration. Am J Respir Cell Mo! Biol, 2021. 65(1): p. 118-121.

## Claims

1. An agent for use in the prevention and/or treatment of lung pathological conditions associated with loss of alveolar epithelial type II and/or type I cells and/or with loss of lung regenerative capacity and/or for use in promoting lung regeneration in a subject with a lung condition, said agent being selected from the group consisting of:
- hsa-miR-124-3p or a primary transcript or a precursor or a synthetic mimic or a functional derivative thereof;
- hsa-miR-5008-5p or a primary transcript or a precursor or a synthetic mimic or a functional derivative thereof;
- hsa-miR-4255 or a primary transcript or precursor or a synthetic mimic or a functional derivative thereof;
- hsa-miR-4691-5p or a primary transcript or a precursor or a synthetic mimic or a functional derivative thereof;
- hsa-miR-4781-5p or a primary transcript or a precursor or a synthetic mimic or a functional derivative thereof;
- hsa-miR-4782-5p or a primary transcript or a precursor or a synthetic mimic or a functional derivative thereof;
- hsa-miR-3928-3p or a primary transcript or a precursor or a synthetic mimic or a functional derivative thereof;
- inhibitor of hsa-miR-183-3p;
- inhibitor of hsa-miR-16-2-3p;
- inhibitor of hsa-mir-147b-3p;
- inhibitor of hsa-miR-1292-3p;
or a combination thereof.

2. The agent for use according to claim 1, wherein the pathological condition or the lung condition is selected from the group consisting of: interstitial lung diseases, preferably idiopathic pulmonary fibrosis, non-specific interstitial pneumonia, desquamative interstitial pneumonia, respiratory bronchiolitis-ILD, cryptogenic organizing pneumonia, acute interstitial pneumonia, chronic obstructive pulmonary diseases, acute and post-acute respiratory distress syndrome, post-acute COVID-19 syndrome.

3. The agent for use according to claim 1 or 2 wherein the pathological condition or the lung condition is idiopathic pulmonary fibrosis, chronic obstructive pulmonary diseases, acute and post-acute respiratory distress syndrome.

4. The agent for use according to any one of previous claims, wherein said inhibitor is a nucleic acid molecule preferably selected from: a single-stranded or double-stranded nucleic acid molecule, an siRNA molecule, an antisense oligonucleotide, derivatives and mixtures thereof.

5. The agent for use according to claim 4, wherein said nucleic acid molecule has sufficient complementarity to hsa-miR-183-3p, hsa-miR-16-2-3p, hsa-mir-147b-3p or hsa-miR-1292-3p to form a hybrid under physiological conditions and/or comprises at least 8 nucleotides complementary to at least one sequence selected from SEQ ID NO: 17-24 or a variant thereof.

6. The agent for use according to any one of previous claims, wherein:
the inhibitor of hsa-miR-183-3p comprises a nucleotide sequence comprising 5'-GTAATTCA-3' and wherein the inhibitor comprises about 8 to about 30 nucleotides in length;
the inhibitor of hsa-miR-16-2-3p comprises a nucleotide sequence comprising 5'-TAATATTG'-3' and wherein the inhibitor comprises about 8 to about 30 nucleotides in length;
the inhibitor of hsa-mir-147b-3p comprises a nucleotide sequence comprising 5'-TCCGCACA-3' and wherein the inhibitor comprises about 8 to about 30 nucleotides in length;
the inhibitor of hsa-miR-1292-3p comprises a nucleotide sequence comprising 5'-GGGGCGCG-3' and wherein the inhibitor comprises about 8 to about 30 nucleotides in length;
and/or the inhibitor comprises at least 1 nucleotide, at least 2 nucleotides, at least 3 nucleotides, at least 4 nucleotides, at least 5 nucleotides, at least 6 nucleotides, at least 7 nucleotides, at least 8 nucleotides, at least 9 nucleotides, at least 10 nucleotides, at least 11 nucleotides, at least 12 nucleotides, at least 13 nucleotides, at least 14 nucleotides, at least 15 nucleotides, at least 16 nucleotides, at least 17 nucleotides, at least 18 nucleotides, at least 19 nucleotides, or at least 20 nucleotides at the 5' and/or at the 3' of said nucleotide sequence.

7. The agent for use according to any one of previous claims, wherein:
the inhibitor of hsa-miR-183-3p has at least 50%, 60%, 70%, 80%, 90%, 95%, 99% or 100% complementarity to a portion of a hsa-miR-183-3p sequence;
the inhibitor of hsa-miR-16-2-3p has at least 50%, 60%, 70%, 80%, 90%, 95%, 99% or 100% complementarity to a portion of a hsa-miR-16-2-3p sequence;
the inhibitor of hsa-mir-147b-3p and/or has at least 50%, 60%, 70%, 80%, 90%, 95%, 99% or 100% complementarity to a portion of a hsa-miR-147b-3p sequence;
the inhibitor of hsa-miR-1292-3p has at least 50%, 60%, 70%, 80%, 90%, 95%, 99% or 100% complementarity to a portion of a hsa-miR-1292-3p sequence.

8. The agent for use according to any one of previous claims, wherein the inhibitor binds to a miRNA comprising a nucleic acid that is at least 50%, 60%, 70%, 80%, 90%, 95%, 99% or 100% identical to any one SEQ ID NOs: 17-24.

9. The agent for use according to any one of previous claims, wherein the inhibitor comprises at least one modified nucleotide, preferably the at least one modified nucleotide is a locked nucleic acid (LNA), an unlocked nucleic acid (UNA), an arabino nucleic acid (ABA), a bridged nucleic acid (BNA), and/or a peptide nucleic acid (PNA) and/or the inhibitor comprises a backbone modification, preferably a phosphorodiamidate morpholino oligomer (PMO) and/or phosphorothioate (PS) modification.

10. A nucleic acid coding for the at least one agent as defined in any of claims 1 to 9 for use in the treatment and/or prevention of lung pathological conditions associated with loss of alveolar epithelial type II and/or type I cells and/or with loss of lung regenerative capacity and/or for use in promoting lung regeneration in a subject with a lung condition, wherein the pathological condition is preferably selected from the group of interstitial lung diseases, preferably idiopathic pulmonary fibrosis, non-specific interstitial pneumonia, desquamative interstitial pneumonia, respiratory bronchiolitis-ILD, cryptogenic organizing pneumonia, acute interstitial pneumonia, chronic obstructive pulmonary diseases, acute and post-acute respiratory distress syndrome, post-acute COVID-19 syndrome.

11. The agent for use according to any one of claims 1-9 or the nucleic acid for use according to claim 10, wherein said agent or nucleic acid is provided naked or within a delivery agent, preferably wherein the delivery agent comprises a vector, preferably a recombinant expression vector or a viral vector, a micelle, an exosome, a lipidoid, a lipoplex, an extracellular vesicle, a synthetic vesicle, a polymeric compound, a peptide, a protein, a cell, a nanoparticle mimic, a nanotube, a conjugate, nanoparticles, microparticles, a liposome or other biological or
synthetic vesicle or material including lipid nanoparticles, polymer-based nanoparticles, polymer-lipid hybrid a nanoparticle, a microparticle, a microsphere, a liposome, a colloidal gold particle, a graphene composite, a cholesterol conjugate, a cyclodextran complexe, a polyethylenimine polymer, a lipopolysaccharide, a polypeptide, a polysaccharide, a lipopolysaccharide, a collagen, a pegylation of viral vehicle, or combinations thereof.

12. A vector, preferably a recombinant expression vector, comprising a coding sequence for the agent as defined in any of claims 1 to 9 or the nucleic acid of claim 10, and/or expressing the agent as defined in any one of claims 1 to 9 or the nucleic acid of claim 10, preferably under the control of a suitable promoter, for use in the treatment and/or prevention of lung pathological conditions associated with loss of alveolar epithelial type II and/or type I cells and/or with loss of lung regenerative capacity and/or for use in promoting lung regeneration in a subject with a lung condition, wherein the pathological condition is preferably selected from the group of interstitial lung diseases, preferably idiopathic pulmonary fibrosis, non-specific interstitial pneumonia, desquamative interstitial pneumonia, respiratory bronchiolitis-ILD, cryptogenic organizing pneumonia, acute interstitial pneumonia, chronic obstructive pulmonary diseases, acute and post-acute respiratory distress syndrome, post-acute COVID-19 syndrome,
preferably the vector is a viral or non-viral vector, preferably the viral vector is selected from adeno-associated virus (AAV) vectors of any capsid serotype, either natural or artificial, lentivirus vectors, adenoviral vector, retroviral vectors, alphaviral vectors, vaccinia virus vectors, herpes simplex virus (HSV) vectors, rabies virus vectors, and Sindbis virus vectors, preferably the adeno-associated virus (AAV) vector is AAV6, AAV6.2 or AAV6.2FF.

13. A host cell transformed with the vector as defined in claim 12 for use in the treatment and/or prevention of lung pathological conditions associated with loss of alveolar epithelial type II and/or type I cells and/or with loss of lung regenerative capacity and/or for use in promoting lung regeneration in a subject with a lung condition, wherein the pathological condition is preferably selected from the group of interstitial lung diseases, preferably idiopathic pulmonary fibrosis, non-specific interstitial pneumonia, desquamative interstitial pneumonia, respiratory bronchiolitis-ILD, cryptogenic organizing pneumonia, acute interstitial pneumonia, chronic obstructive pulmonary diseases, acute and post-acute respiratory distress syndrome, post-acute COVID-19 syndrome.

14. A recombinant adeno-associated virus (rAAV) particle comprising a nucleic acid encoding the at least one agent as defined in any one of claims 1-9, preferably the particle comprises a capsid derived from adeno-associated vectors AAV6, AAV6.2, AAV6.2FF, AAV8, AAV1, AAV2, AAV5, or AAV9, preferably wherein the nucleic acid is operably linked to a viral promoter or a tissue specific promoter for use in the treatment and/or prevention of lung pathological conditions associated with loss of alveolar epithelial type II and/or type I cells and/or with loss of lung regenerative capacity and/or for use in promoting lung regeneration in a subject with a lung condition, wherein the pathological condition is preferably selected from the group of interstitial lung diseases, preferably idiopathic pulmonary fibrosis, non-specific interstitial pneumonia, desquamative interstitial pneumonia, respiratory bronchiolitis-ILD, cryptogenic organizing pneumonia, acute interstitial pneumonia, chronic obstructive pulmonary diseases, acute and post-acute respiratory distress syndrome, post-acute COVID-19 syndrome.

15. A pharmaceutical composition for use in the treatment and/or prevention of lung pathological conditions associated with loss of alveolar epithelial type II and/or type I cells and/or with loss of lung regenerative capacity and/or for use in promoting lung regeneration in a subject with a lung condition, wherein the pathological condition is preferably selected from the group of interstitial lung diseases, preferably idiopathic pulmonary fibrosis, non-specific interstitial pneumonia, desquamative interstitial pneumonia, respiratory bronchiolitis-ILD, cryptogenic organizing pneumonia, acute interstitial pneumonia, chronic obstructive pulmonary diseases, acute and post-acute respiratory distress syndrome, post-acute COVID-19 syndrome, said pharmaceutical composition comprising an agent as defined in any one of claims 1-9 or 11, or the nucleic acid as defined in claim 10 or 11, or the vector as defined by claim 12, or the host cell as defined in claim 13, or a recombinant adeno-associated virus (rAAV) particle as defined in claim 14 and at least one pharmaceutically acceptable vehicle and/or excipient.
